# FASCICULE DE BREVET EUROPEEN

(11) **EP 3 322 689 B1**
(45) Date de publication et mention de la délivrance du brevet: **21.11.2018**
(21) Numéro de dépôt: 17745416.2
(22) Date de dépôt: 30.06.2017
(51) Int. Cl.: C07C 67/03, C07C 69/52, C10L 1/02, C11C 3/00

(54) **PROCÉDÉ DE PRODUCTION D'ESTERS D'ACIDES GRAS ET DE GLYCÉROL À BASSE TEMPÉRATURE**
VERFAHREN ZUR HERSTELLUNG VON FETTSÄUREESTERN UND GLYCERIN BEI NIEDRIGER TEMPERATUR
METHOD FOR PRODUCING FATTY ACID ESTERS AND GLYCEROL AT A LOW TEMPERATURE

(30) Priorité: 01.07.2016 FR 1656335
(43) Date de publication de la demande: 23.05.2018
(73) Titulaire: Easyl, 74240 Gaillard (FR)
(72) Inventeur: LACOSTE, François, 92200 Neuilly Sur Seine (FR); THIEL, Julien, 74930 Arbusigny (FR); LAIR, Valentin, 52000 Chaumont (FR); HALLOUMI, Samy, 74800 La Roche Sur Foron (FR)
(74) Mandataire: Jacobacci Coralis Harle
(86) Numéro de dépôt international: PCT/FR2017/051778
(87) Numéro de publication internationale: WO 2018/002559

(56) Documents cités:
- WO-A2-2009/029898
- US-A1- 2015 336 067
- US-B1- 8 580 119

## Description

### DOMAINE TECHNIQUE AUQUEL SE RAPPORTE L'INVENTION

La présente invention concerne de manière générale la production d'esters alkyliques d'acides gras.

Elle concerne plus particulièrement un procédé de production à basse température, à savoir inférieure ou égale à 50°C, d'esters d'acide gras et de glycérol à partir d'une huile végétale ou animale et d'un monoalcool aliphatique au sein d'un broyeur tridimensionnel à microbilles.

### ARRIERE-PLAN TECHNOLOGIQUE

Les esters alkyliques d'acides gras sont actuellement utilisés dans de nombreuses applications, comme carburants diesel, fuels domestiques, solvants écologiques, composés de base pour la fabrication de sulfonates d'alcools gras, d'amides, de dimères d'esters, etc.

En particulier, l'une des applications majeures des esters alkyliques d'acide gras concerne la production de biodiesel destiné à être incorporé dans du carburant gazole. Le biodiesel est notamment un biocarburant d'origine agricole qui constitue une alternative au carburant d'origine fossile usuel.

De façon connue, le biodiesel ou les esters alkyliques d'acide gras sont le produit de la réaction de transestérification entre des triglycérides (TG) et un monoalcool, comme le méthanol, en présence d'un catalyseur homogène ou hétérogène. Un second produit, le glycérol, est également produit lors de cette réaction. Selon la nature de l'huile engagée au départ, le glycérol peut représenter de 10 à 15% en masse des produits formés. Ce glycérol peut être valorisé dans diverses applications, mais nécessite au préalable d'être purifié (élimination métaux, sels, eau).

La réaction de transestérification peut être représentée de la façon suivante lorsque l'alcool de départ est le méthanol : 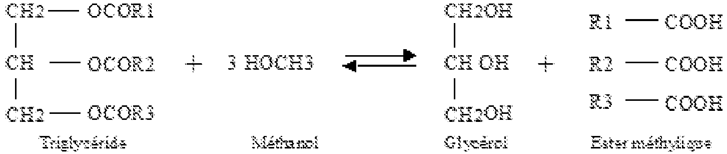 dans laquelle R1, R2 et R3 correspondent à des chaînes hydrocarbonées d'acides gras.

Ainsi, la molécule de triglycéride est mise en réaction avec trois molécules d'alcool méthylique (ou méthanol) pour produire une molécule de glycérol (trialcool liquide donnant par estérification des acides gras) et trois acides appelés esters méthyliques (biodiesel brut). Parmi les sources de triglycérides, on retrouve les huiles végétales ou animales ou encore les huiles usagées de cuisson.

Typiquement, le monoalcool peut être choisi parmi le méthanol, l'éthanol ou encore le butanol ; le méthanol étant cependant l'alcool réactif le plus répandu du fait de son bas prix.

Ensuite le biodiesel brut obtenu est raffiné, puis utilisé comme carburant.

A titre d'exemple, en France, selon les arrêtés du 30 juin 2014 et du 31 décembre 2014, les esters méthyliques d'acides gras ou EMAG peuvent être incorporés au maximum à 8% en volume de gazole. Les exigences requises sont notamment définies dans la norme NF EN 14214 :2013. Ainsi, le biodiesel doit contenir peu de diglycérides (teneur maximale de 0,20% molaire), de monoglycérides (teneur maximale de 0,20% molaire) et peu de glycérol libre (teneur maximale de 0,02% molaire). De plus, afin de renforcer les exigences relatives à la tenue au froid des gazoles à base de biodiesel, la teneur en esters saturés doit être en masse par rapport à la masse totale inférieure à 30% en été et à 16% en hiver. En effet, plus la teneur en esters saturés dans un carburant est importante et moins sa tenue au froid est bonne.

Cependant, la réaction chimique de transestérification décrite ci-dessus présente deux désavantages : elle est lente et équilibrée, à savoir lorsque l'huile et l'alcool se sont transformés en biodiesel et glycérol, le biodiesel et le glycérol peuvent à nouveau se transformer en alcool et en huile.

C'est pourquoi, afin de pallier ces inconvénients, il est généralement considéré comme nécessaire d'utiliser un catalyseur et de travailler sous pression et à température élevée. Egalement, une façon de pousser la conversion de l'huile vers la formation d'ester alkylique (méthylique) est de travailler avec un fort excès de monoalcool par rapport à la stoechiométrie et d'effectuer deux étapes réactionnelles entre lesquelles on élimine le glycérol produit de façon à déplacer l'équilibre vers la production d'ester alkylique.

De nombreux procédés de fabrication d'esters alkyliques d'acides gras en continu ou en discontinu ont déjà été développés dans l'art antérieur.

Une première approche consiste à utiliser les voies classiques de la catalyse homogène avec des catalyseurs solubles dans le milieu réactionnel, comme la soude ou le méthylate de sodium, en faisant réagir une huile neutre et un alcool (méthanol). On peut citer comme représentatif de ce type de procédé, le procédé décrit dans la demande de brevet EP-0.523.767, avec mise en oeuvre continue d'un catalyseur homogène basique.

Ce type de procédé présente cependant plusieurs inconvénients. Une fois la réaction terminée, il faut neutraliser l'excès de catalyseur homogène présent essentiellement dans la phase glycérine sous forme d'alcoolates et de savons, puis éliminer l'eau et le monoalcool (méthanol) par évaporation. Le monoalcool (méthanol) évaporé doit souvent être distillé. Pour la fraction ester, on élimine les traces de composés alcalins par lavage à l'eau et séchage.

Le document US 7.145.026 décrit un procédé pour fabriquer des esters méthyliques d'acides gras en continu à une température allant de 80 à 180°C faisant également intervenir un catalyseur homogène. En particulier, selon un mode de réalisation particulier, ce procédé consiste notamment à faire réagir le méthanol pré-mélangé avec un catalyseur caustique, tel que NaOH, avec des TG dans un réacteur à écoulement piston formé de tubes de cuivre enroulés par exemple d'environ 2 mètres (en général allant de 3 à 9 m pour un diamètre moyen de 15 cm) pendant un temps de séjour allant de 9 à 16 secondes et une température allant de 50°C à 87°C. Plus la température est augmentée et plus le taux de conversion en esters est élevé : il est par exemple de 78% pour un temps de séjour de 9 secondes à 86,8°C et de 43% pour un temps de séjour de 9 secondes à 65 °C. Il est également indiqué que le taux de conversion est augmenté en augmentant la pression, par exemple de 1 bar à 15 bars ou encore le temps de séjour. La montée en température et en pression peut être réalisée dans des dispositifs appropriés placés en amont du réacteur. Afin d'obtenir des esters méthyliques d'acides gras, les produits de la réaction (glycérol, catalyseur, esters méthyliques) sont séparés dans un séparateur prévu à cet effet (séparation des produits par chauffage, puis les esters méthyliques sont nettoyés à l'eau).

Par conséquent, même si les taux de conversion sont relativement élevés, ils restent loin des taux optimaux de 90% ou plus, et ce procédé présente également l'inconvénient de devoir éliminer le catalyseur des produits de la réaction. En plus, il nécessite des étapes de chauffage et de montée en pression qui complexifient la mise en oeuvre de ce procédé.

Une deuxième approche met en oeuvre des catalyseurs hétérogènes, à savoir insolubles dans le milieu réactionnel.

Le document EP 0 924 185 décrit notamment un procédé de fabrication d'esters alkyliques dérivés d'huiles végétales comprenant trois étapes :
- une première étape (a) consiste à faire réagir une huile végétale avec un excès de monoalcool en présence d'un catalyseur hétérogène, suivi d'une élimination du monoalcool en excès et d'une séparation de la glycérine, de sorte à obtenir un ester brut contenant des monoglycérides résiduels ;
- une seconde étape (b), dans laquelle on soumet l'ester brut ainsi obtenu à une réaction de ré-estérification des mono-glycérides résiduels vers des di- et triglycérides, en présence d'un catalyseur hétérogène ; et
- une troisième étape (c), dans laquelle on procède à une évaporation sous pression réduite de l'ester avec recyclage du résidu d'évaporation vers l'huile de départ de l'étape (a).

L'inconvénient principal d'un tel procédé est le coût économique très important que représente la distillation sous vide de toute la production. De plus, la présence d'un recyclage représente également un surcoût. Enfin, des expériences montrent que, même sous pression très réduite, la température de fond de la colonne d'évaporation de l'ester est importante, ce qui entraîne un risque sérieux de dégradation du résidu. Ce dernier ne peut donc être totalement recyclé, il doit périodiquement être purgé, ce qui pénalise le rendement du procédé.

Le document FR 2 838 433 décrit également un procédé de production d'esters alkyliques d'acides gras et de glycérol en présence d'un catalyseur hétérogène, l'aluminate de zinc. Ce procédé consiste en une succession de trois réactions équilibrées se déroulant en parallèle :
- une huile, telle qu'une huile de colza, de palme, de tournesol, etc. (représentant 20 à 80%, de préférence 45 à 55% en masse) est mise à réagir avec une molécule de monoalcool, comme le méthanol, pour donner une molécule d'ester alkylique et un diglycéride ;
- le diglycéride ainsi obtenu (représentant 20 à 80%, de préférence 45 à 55% en masse) est mis à réagir avec une molécule de monoalcool (méthanol) pour donner une molécule d'ester alkylique (méthylique) et un monoglycéride ;
- le monoglycéride ainsi obtenu est mis à réagir avec une molécule de monoalcool (méthanol) pour donner une molécule d'ester alkylique (méthylique) et une molécule de glycérine.

La réaction se fait en général dans plusieurs réacteurs en lit fixe successifs opérés en écoulement ascendant et en phase liquide, chacun des réacteurs étant alimenté par un mélange d'huile de colza et de monoalcool (méthanol) (premier réacteur) ou majoritairement d'ester alkylique (méthylique) et de monoalcool (méthanol) (second réacteur et éventuellement les réacteurs suivants). Les réactions sont opérées à une température allant de 180°C à 220°C sous une pression de 30 bars à 80 bars. Entre les réactions, le monoalcool en excès est éliminé par évaporation afin d'être recyclé et le produit obtenu est mis à décanter dans un ballon décanteur afin de séparer les phases riches en ester alkylique de la phase riche en glycérol.

En sortie de réacteur, on obtient l'ester méthylique et un coproduit de la réaction, le glycérol, ainsi que le méthanol en excès.

Par conséquent, ce procédé présente l'inconvénient d'être très complexe, de nécessiter l'emploi de nombreux dispositifs positionnés de façon successive (réacteurs/évaporateurs/condensateurs/décanteurs, etc.). En outre, il nécessite de travailler sous hautes températures (180°C à 220°C) et sous hautes pressions (30 bars à 80 bars).

Le document US 2007/0260079 décrit un procédé de fabrication de biodiesel pouvant faire intervenir un catalyseur homogène, un catalyseur hétérogène ou les deux.

En particulier, ce procédé consiste :
- à faire réagir les TG, l'alcool (méthanol), le catalyseur dans une première zone de réaction formée d'un mélangeur suivi d'un réacteur tubulaire (durée de séjour 20 à 30 secondes sous une pression de 3,5 à 20 bars et une température allant de 70 à 200°C) afin de former un premier mélange intermédiaire qui comprend le produit ester d'acide gras trans-estérifié, l'alcool qui n'a pas régi, les TG qui n'ont pas réagi ou ont partiellement réagi ;
- à éliminer le glycérol et l'alcool qui n'a pas réagi de ce mélange intermédiaire grâce notamment à un hydrocyclone qui permet de séparer la phase plus lourde, à savoir le glycérol, des esters méthylique d'acides gras formés compris dans un deuxième mélange intermédiaire ;
- à amener ce deuxième mélange intermédiaire vers une seconde zone de réaction comprenant également un mélangeur suivi d'un réacteur tubulaire ;
- ajouter à cette seconde zone de réaction de l'alcool et le catalyseur (au niveau du mélangeur) afin de former un troisième mélange intermédiaire, qui une fois amené au niveau du réacteur (temps de séjour de l'ordre de 30 à 60 secondes sous une pression de 10 à 27,5 bars et une température allant de 70 à 200°C) permettra de former des esters alkyliquesd'acides gras et du glycérol ;
- à séparer l'alcool, le glycérol et les esters alkyliques d'acides gras grâce à un hydrocyclone.

Par conséquent, ce procédé présente également l'inconvénient de nécessiter l'emploi de nombreux dispositifs (mélangeur/réacteur et hydrocyclone) positionnés successivement afin de réaliser la réaction de transestérification et de nécessiter des étapes de chauffage et de mise sous pression.

On connaît également de l'état de la technique le document US2015/0336067 qui décrit la fabrication de biodiésel à partir d'une huile végétale, de méthanol et d'un catalyseur. Ces matières premières sont placées dans un réservoir qui est relié à un dispositif à vibration qui permettait la réaction de transestérification.

US 2015/336067 divulgue une procédé de préparation du biodiesel, dans lequel un huile végétale est émulsionné avec du méthanol et un catalyseur. Ensuite, cette émulsion est introduite dans un réacteur où la réaction de transestérification a lieu à une température de 20°C.

En résumé, pour fabriquer le biodiesel, il est à l'heure actuelle nécessaire de recourir à de si nombreuses étapes que seules de grandes unités sont en général capables d'être économiquement rentables.

Il existe ainsi un réel besoin d'un nouveau procédé de fabrication d'esters alkyliques d'acides gras qui permettent la fabrication de tels composés de manière simple et économique, par exemple en ayant un nombre plus réduit d'étapes, en utilisant moins de dispositifs de mises en oeuvre (réacteur, décanteur, évaporateur, condenseur, etc. placés successivement), tout en étant rapide (temps de séjour réduit).

Il existe également un besoin de fournir un nouveau procédé dans lequel la fabrication de ces esters alkyliques d'acides gras ne nécessite pas de chauffage particulier ou de mise sous pression pouvant augmenter les coûts de fabrication.

Le but de la présente invention est, par conséquent, de proposer un nouveau procédé de fabrication d'esters alkyliques d'acides gras évitant au moins en partie les inconvénients précités.

### OBJET DE L'INVENTION

A cet effet, la présente invention a pour objet un procédé de production d'esters alkyliques d'acides gras et de glycérol mettant en oeuvre un ensemble de réactions de transestérification entre au moins une huile végétale ou animale et au moins un monoalcool aliphatique comprenant les étapes suivantes :
(A) l'introduction dans un broyeur tridimensionnel à microbilles, séparément ou en mélange, d'au moins une huile végétale et/ou animale, d'au moins un monoalcool aliphatique et d'au moins un catalyseur hétérogène et/ou homogène pour former un mélange initial ;
(B) le broyage dudit mélange initial à une température inférieure ou égale à 50°C, de préférence inférieure ou égale à 25°C, dans un broyeur tridimensionnel à microbilles, pendant un temps de séjour inférieur ou égal à 5 minutes, de préférence allant de 5 à 30 secondes et typiquement allant de 5 à 15 secondes ;
(C) la récupération en sortie du broyeur tridimensionnel d'un mélange final comportant au moins des esters alkyliques d'acides gras, du glycérol, le catalyseur et le monoalcool aliphatique n'ayant pas réagi; et
(D) la séparation de ce mélange final d'une première phase comprenant les esters alkyliques d'acides gras, d'une deuxième phase comprenant le glycérol et le monoalcool aliphatique n'ayant pas réagi et le catalyseur.

Pour le reste de la description, à moins qu'il n'en soit spécifié autrement, l'indication d'un intervalle de valeurs « de X à Y » ou « entre X et Y », dans la présente invention, s'entend comme incluant les valeurs X et Y.

Selon l'invention, une température inférieure ou égale à 50°C inclut les valeurs suivantes ou tout intervalle entre ces valeurs : 50 ; 49 ; 48 ; 47 ; 46 ; 45 ; 44 ; 43 ; 42 ; 41 ; 40 ; 39 ; 38 ; 37 ; 36 ; 35 ; 34 ; 33 ; 32 ; 31 ; 30 ; 29 ; 28 ; 27 ; 28 ; 27 ; 26 ; 25 ; 24 ; 23 ; 22 ; 21 ; 20 ; 19 ; 18 ; 17 ; 16 ; 15 ; 14 ; 13 ; 12 ; 1 1 ; 10 ; etc.

Egalement, par temps de séjour inférieur ou égal à 5 minutes, on entend un temps de séjour inférieur ou égal aux valeurs suivantes ou à tout intervalle compris entre ces valeurs : 5 min ; 4 min ; 3 min ; 2 min ; 1 min ; 50 sec ; 45 sec ; 40 sec ; 35 sec ; 30 sec ; 25 sec ; 20 sec ; 15 sec ; 10 sec ; 8 sec ; 5 sec ; etc. Généralement, le temps de séjour du mélange initial correspond au débit utilisé lors du procédé sur le volume du mélange initial au sein de ce broyeur (le volume du mélange initiale étant égal au volume du broyeur - le volume pris par les microbilles).

D'autres caractéristiques non limitatives et avantageuses du procédé de production d'esters alkyliques d'acides gras conforme à l'invention, prises individuellement ou selon toutes les combinaisons techniquement possibles, seront décrites ci-après.

L'invention a également pour objet un produit susceptible d'être obtenu par le procédé susmentionné, comprenant au moins des esters d'acides gras, du glycérol, un catalyseur et un monoalcool, caractérisé en ce qu'il comporte moins de 30 %, en masse, par rapport à la masse totale du produit, dudit monoalcool.

### DESCRIPTION DETAILLEE D'UN EXEMPLE DE RÉALISATION

La description qui va suivre en regard des dessins annexés, donnés à titre d'exemples non limitatifs, fera bien comprendre en quoi consiste l'invention et comment elle peut être réalisée.

Sur les dessins annexés :
- La figure 1 est une vue schématique des différentes étapes du procédé selon un mode de réalisation particulier de l'invention. Sur cette figure, sont également représentées, en traits interrompus, des variantes de réalisation de l'invention ;
- la figure 2 représente une vue en coupe selon l'axe longitudinal XX d'un broyeur tridimensionnel à microbilles en phase humide, selon une variante de réalisation convenant pour la mise en oeuvre du procédé selon l'invention ;
- la figure 3 représente des vues en coupe selon l'axe XX et l'axe AA, de variantes de broyeurs tridimensionnels à microbilles en phase humide selon la figure 2 dans lesquels : (a) l'agitateur est à disques, (b) l'agitateur comporte des doigts et (c) la chambre de broyage est annulaire ;
- la figure 4 est un chromatogramme issu de l'analyse HPLC d'une huile végétale de tournesol avant broyage et de l'analyse HPLC de l'huile de tournesol transformée après broyage obtenue à l'exemple n°3 selon le procédé de l'invention.

Egalement, le tableau 3 ci-dessous dans la partie exemple est un tableau de résultats des pourcentages de conversion obtenus considérant différentes huiles et en faisant varier les conditions expérimentales, telles que le ratio molaire, la teneur massique de catalyseur, le débit de passage, le diamètre des billes ; etc

La Demanderesse s'est attachée au développement d'un nouveau procédé de fabrication d'esters alkyliques d'acides gras adapté à une mise en oeuvre à une échelle industrielle, qui soit moins onéreux et simplifié par rapport aux procédés traditionnels décrits ci-dessus.

En particulier, la Demanderesse a découvert de manière surprenante que l'emploi d'un broyeur tridimensionnel à billes, usuellement utilisé dans l'industrie, telle que l'industrie pharmaceutique pour réduire la taille de particules ou pour réaliser des mélanges homogènes entre deux composés, permettrait de réaliser une réaction de chimie organique et de synthétiser des esters alkyliques d'acides gras à partir d'une huile végétale et/ou animale et d'un monoalcool et ce à un taux de conversion très élevé, pour un seul passage, en général supérieur ou égal à 95%, de préférence supérieur ou égal à 98%.

L'installation mise en oeuvre pour la réalisation de ce procédé est en effet très simple et peut être composée seulement d'un broyeur tridimensionnel à microbilles suivi d'un séparateur. De plus, cette installation peut être de dimension variable et être facilement implantée près des sites de récupération d'huiles usagées.

De plus, comme cela sera démontré dans la partie expérimentale ci-dessous, l'emploi d'un tel broyeur permet de réaliser de manière simple et non onéreuse un biodiesel et ce en un temps très court sans nécessiter d'étapes de chauffage et/ou de mise sous pression particulières. En effet, le travail s'effectue généralement à température et pression ambiantes. En outre, le procédé de l'invention requiert de préférence un ratio molaire « monoalcool/huile végétale et/ou animale » proche de la stoechiométrie sans qu'il soit de plus nécessaire d'éliminer le monoalcool au cours du procédé ou encore d'en rajouter afin de déplacer l'équilibre de la réaction de transestérification.

Egalement, ce procédé permet de limiter la consommation d'eau, notamment lorsque le catalyseur utilisé est un catalyseur homogène, par rapport aux procédés de l'art antérieur.

Le procédé selon l'invention présente également l'avantage de pouvoir être mis en oeuvre de façon continue. Or, ces caractéristiques sont importantes pour une application à l'échelle industrielle.

Ainsi, telle que mentionnée ci-dessus, la présente invention porte sur un procédé de production d'esters alkyliques d'acides gras et de glycérol mettant en oeuvre un ensemble de réactions de transestérification entre au moins une huile végétale et/ou animale et au moins un monoalcool aliphatique comprenant les étapes suivantes :
(A) l'introduction dans un broyeur tridimensionnel à microbilles séparément ou en mélange d'au moins une huile végétale et/ou animale, d'au moins un monoalcool aliphatique et d'au moins un catalyseur hétérogène et/ou homogène pour former un mélange initial ;
(B) le broyage dudit mélange initial à une température inférieure ou égale à 50°C, de préférence inférieure ou égale à 25°C, dans un broyeur tridimensionnel à microbilles, pendant un temps de séjour inférieur ou égal à 5 minutes, de préférence allant de 5 à 30 secondes et typiquement allant de 5 à 15 secondes ;
(C) la récupération en sortie du broyeur tridimensionnel d'un mélange final comportant au moins des esters alkyliques d'acides gras, du glycérol, le catalyseur et le monoalcool aliphatique n'ayant pas réagi; et
(D) la séparation de ce mélange final d'une première phase comprenant les esters alkyliques d'acides gras et d'une deuxième phase comprenant le glycérol, le monoalcool aliphatique n'ayant pas réagi et le catalyseur.

De préférence, le procédé comprend en outre une étape (E) d'élimination du monoalcool avant et/ou après l'étape (D) de séparation.

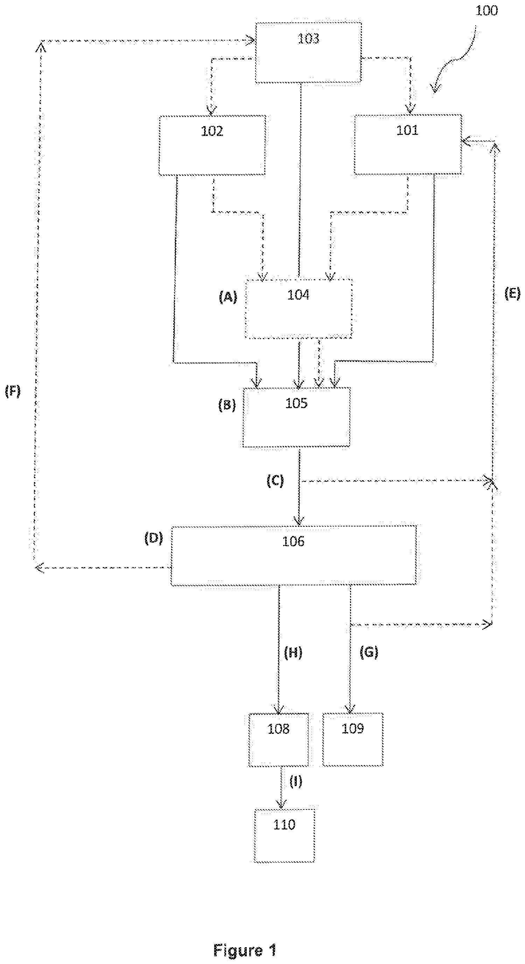

En se référant à la figure 1, le procédé 100 selon l'invention va désormais être décrit plus explicitement ci-dessous.

Tout d'abord, la fabrication des esters alkyliques d'acides gras selon l'invention peut comprendre initialement (A) une étape de mélange d'au moins une huile végétale et/ou animale avec au moins un monoalcool aliphatique, par exemple par tout moyen mécanique.

Pour cela, le monoalcool peut être stocké dans une première cuve 101, l'huile végétale et/ou animale dans une deuxième cuve 102 et le catalyseur dans une troisième cuve 103.

Selon un mode de réalisation particulier, le catalyseur peut être pré-mélangé et mis en suspension avec le monoalcool (par exemple, lorsque le catalyseur utilisé est un catalyseur homogène). Il peut également être pré-mélangé et mis en suspension avec l'huile (par exemple, lorsque le catalyseur utilisé est un catalyseur hétérogène), ce pré-mélange étant placé dans la cuve 102.

L'huile végétale et/ou animale contenant éventuellement le catalyseur, le monoalcool contenant éventuellement le catalyseur et le catalyseur pouvant être également introduits directement dans le broyeur à microbilles 105.

Le monoalcool convenant pour la présente invention peut être par exemple choisi parmi tous les monoalcools aliphatiques primaires et secondaires ayant en général 1 à 8 atomes de carbone. Le monoalcool préféré pour le procédé de transestérification de la présente invention est par exemple choisi parmi un ou plusieurs des monoalcools suivants : le méthanol, l'éthanol, le propanol, l'isopropyle ou encore le butanol ; le monoalcool particulièrement préféré étant le méthanol ou l'éthanol et est typiquement le méthanol.

En particulier, le méthanol convient pour réaliser le procédé selon l'invention du fait de son faible coût, qu'il est capable de réagir rapidement et que de nombreux catalyseurs homogènes, tels que NaOH, s'y dissolvent facilement.

Selon l'invention, l'huile végétale et/ou animale correspond à des substances naturelles ou élaborées, d'origine animale ou végétale, contenant majoritairement des triglycérides (TG), à savoir au moins 50% en masse par rapport à la masse totale de l'huile, de préférence au moins 60% et typiquement au moins 70%. En particulier, les TG correspondent à du glycérol dont tous les groupes hydroxy sont substitués par des acides gras présentant indépendamment une chaîne linéaire ou ramifiée, saturée ou insaturée, ayant de préférence de 4 à 24 atomes de carbone et en particulier de 12 à 18 atomes de carbone par groupe d'acides gras.

De préférence, l'huile végétale et/ou animale peut être toutes huiles connues de l'homme du métier, et être par exemple choisies parmi une ou plusieurs des huiles suivantes : l'huile de maïs, l'huile de lin, l'huile de colza, l'huile d'olive, l'huile de palme, l'huile de canola, l'huile de coprah, l'huile de soja, l'huile de graine de coton, l'huile d'arachide, de pourghère, l'huile de carthame, l'huile de tournesol, l'huile de coprah, l'huile de babassu, l'huile de ricin, et toutes huiles issues par exemple du tournesol ou du colza par modification génétique ou hybridation ou encore provenant d'algues, le suif, le lard, les huiles de poisson, de phoque, le saindoux ou encore une huile usagée, telle qu'une huile de friture usagée, les graisses issues du traitement des eaux usées et même des graisses de volailles.

Il est en effet possible d'utiliser les huiles usagées, comme les huiles de friture, d'équarrissage, etc. car les esters fabriqués à partir de certains alcools comme l'alcool éthylique, isopropylique ou butylique, permettent de gagner plus de 10°C en point d'écoulement et par conséquent, d'utiliser au départ des huiles plus saturées. Cependant, lorsqu'on utilise des huiles de friture, qui constituent une matière première très bon marché pour produire un biodiesel, il est nécessaire d'éliminer du mélange réactionnel les polymères d'acides gras afin que le mélange d'esters réponde aux spécifications de la norme EN 14214. L'objectif reste de maintenir l'indice d'acide de l'huile de préférence inférieur à 0.5mg KOH/g. En effet, la présence d'acides gras dans le milieu peut entraîner des réactions d'estérification qui produisent trop d'eau, ce qui peut réduire la pureté de la phase glycérine coproduite.

Parmi les huiles utilisées, on peut encore indiquer des huiles partiellement modifiées par exemple par polymérisation ou oligomérisation, comme par exemple, les "standolies" d'huile de lin, de tournesol et les huiles végétales soufflées. Les huiles utilisées sont neutres ou acides, vierges ou recyclées.

Selon une caractéristique de l'invention, il est possible de faire passer au préalable (à savoir avant l'étape de mélange (B)), l'huile selon l'invention dans un sécheur sous vide de façon à obtenir une teneur en eau inférieure à 700 ppm en masse, également appelée « huile sèche ».

Le ou les catalyseurs convenant pour le procédé de l'invention peuvent être choisis parmi un catalyseur homogène liquide et/ou un catalyseur hétérogène solide.

A titre d'exemple, un catalyseur homogène peut correspondre à des composés de métaux alcalins ou alcalino-terreux solubles dans le monoalcool, tels que des hydroxydes et méthoxydes des métaux alcalins et alcalinoterreux et des acides, tels que H₂SO₄.

De préférence, on peut citer les catalyseurs homogènes suivants : NaOH, LiOH, KOH, K₂CO₃, KNO₃, KF ou LiNO₃.

Les catalyseurs selon l'invention peuvent être des catalyseurs hétérogènes solides, tels que décrits par Marinkovich et al dans les articles « Calcium oxide as a promising heterogeneous catalyst for biodiesel production: Current state and perspectives » (Renewable and Sustainable Energy Reviews 56 (2016)1387-1408) et ceux décrits dans le brevet US-7.145.026.

De préférence, le catalyseur hétérogène solide convenant pour le procédé selon l'invention est choisi parmi un ou plusieurs des composés suivants : l'oxyde de calcium, l'oxyde de zinc, un mélange d'oxyde de zinc et d'alumine, un aluminate de zinc répondant à la formule ZnAl₂O₄, (ZnO)ₓ (Al₂O₃)_{y} où x et y sont compris chacun entre 0 et 2, le zincate de calcium, le calcium diglycéroxyde. Préférentiellement, le catalyseur hétérogène solide est choisi parmi : l'oxyde de calcium, le zincate de calcium, le calcium diglycéroxyde et ou un de leurs mélanges.

En particulier, le catalyseur selon l'invention est le zincate de calcium de formule Ca[Zn(OH)₃]₂.2H₂O, préalablement calciné à une température allant de 400°C à 600°C. En général, la calcination a lieu en présence d'air ou sous atmosphère d'au moins un gaz neutre. De préférence, le zincate de calcium présente une surface spécifique supérieure ou égale à 10 m²/g.

En particulier, le catalyseur de zincate de calcium peut être préparé conformément à la demande de brevet portant le numéro de dépôt FR 15 52884 et le catalyseur à base d'oxyde de zinc peut être préparé conformément à la demande de brevet portant le numéro de publication FR 2 962 727 ou FR 2 752 242.

Il convient de noter que le catalyseur peut être une combinaison de catalyseurs homogènes et hétérogènes.

Pour réaliser l'étape (A) du procédé, le monoalcool, l'huile végétale et/ou animale contenant le catalyseur (comme cela est représenté selon un mode de réalisation de la figure 1) sont amenés, généralement sans étape de chauffage particulière (à savoir à température ambiante entre 15-25°C), par exemple via des conduits spécifiques, vers un mélangeur 104 ayant pour fonction d'homogénéiser les différents constituants afin de former le mélange initial selon l'invention. Ce mélangeur 104 est bien connu de l'homme du métier et ne sera pas décrit plus en détail ci-après. A titre d'exemple, on peut citer les mélangeurs à pales.

En variante, la préparation du mélange initial peut être effectuée préalablement indépendamment du procédé de l'invention et introduite telle quelle dans le broyeur 105 ou réalisée directement dans le broyeur 105.

En général, le catalyseur présent dans le mélange initial représente, en masse, par rapport à la masse totale d'huile végétale et/ou animale moins de 4%, de préférence de 1 à 4%.

Au sens de l'invention, une teneur massique de 4% ou moins inclut les valeurs suivantes ou tout intervalle compris entre ces valeurs : 4 ; 3,5 ; 3 ; 2,5 ; 2 ; 1,5 ; 1 ; 0,5, etc.

De préférence, le rapport molaire « monoalcool/huile végétale et/ou animale » dans le mélange initial est inférieur ou égal à 15, de préférence inférieur ou égal à 6 et mieux inférieur ou égal à 4. Typiquement, le rapport molaire « monoalcool/huile végétale et/ou animale » dans le mélange initial est supérieur ou égal à 3, valeur stoechiométrique de la réaction.

Au sens de l'invention, un ratio molaire inférieur ou égal à 15 inclut les valeurs suivantes ou tout intervalle entre ces valeurs : 15 ; 14 ; 13 ; 12 ; 11 ; 10 ; 9;8;7;6;5;4;3.

Egalement, le rapport massique « catalyseur / huile + monoalcool » dans le mélange initial est de préférence inférieur ou égal à 5%, de préférence inférieur ou égal à 3% et typiquement inférieur ou égal à 2%.

Le mélange initial ne comprend en outre de l'eau qu'à l'état de trace, à savoir le mélange initial a généralement une teneur en eau inférieure ou égale à 1500ppm.

Le mélange initial, lorsqu'il est préparé préalablement à son introduction dans le broyeur 105, est acheminé de manière homogène vers le broyeur tridimensionnel à microbilles 105 selon l'invention de sorte à procéder à l'étape de broyage (B).

Cet acheminement s'effectue également de préférence sans chauffage particulier, à savoir à une température inférieure ou égale à 50°C, de préférence à une température inférieure ou égale à 35°C et typiquement à une température inférieure ou égale à 25°C. Il peut s'effectuer par exemple via des conduits prévus à cet effet.

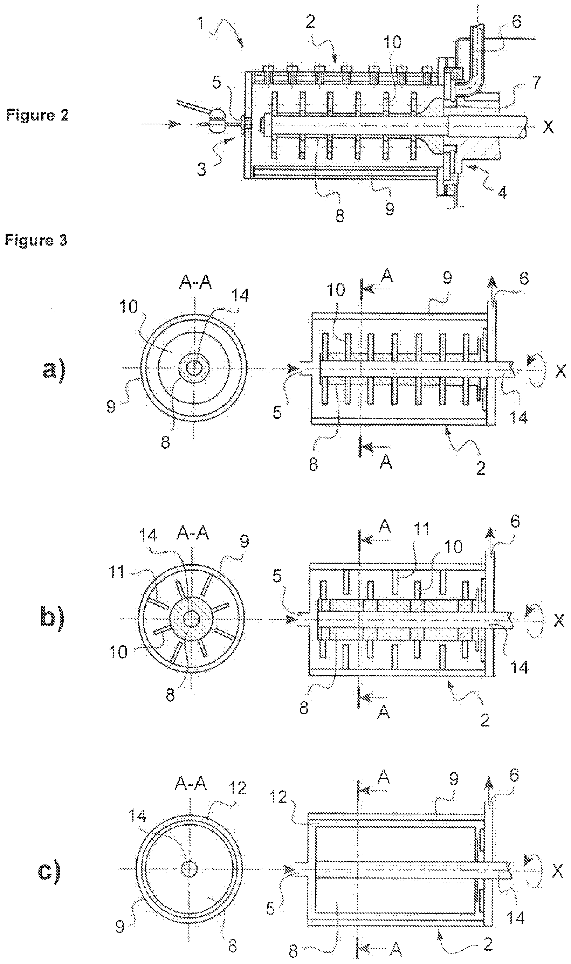

Pour mieux faire comprendre le procédé objet de l'invention, un broyeur tridimensionnel à microbilles susceptible de permettre la production d'ester alkyliques d'acides gras et de glycérol, et faisant ainsi partie de l'invention, va être décrit ci-dessous en se référant aux figures 2 et 3.

Tel qu'illustré sur la figure 2, un broyeur tridimensionnel à microbilles 1 comprend au moins :
- une chambre de broyage 2 stationnaire de forme générale cylindrique s'étendant selon un axe longitudinal XX, ladite chambre 2 étant remplie au moins, en partie, par lesdites microbilles (non représentées) et comprend: à une première extrémité 3 au moins une entrée 5 servant à introduire ledit mélange initial, et à une seconde extrémité 4, une sortie 6 comportant un moyen de séparation 7 apte à n'évacuer que le mélange final ainsi formé (produit formé dans le broyeur comprenant essentiellement les esters alkyliques d'acides gras, le glycérol, le catalyseur et le monoalcool aliphatique n'ayant pas réagi) dans ladite chambre 2; et
- un agitateur 8, disposé dans la chambre de broyage 2 stationnaire, se présentant sous forme d'une tige allongée selon l'axe longitudinal XX, ledit agitateur 8 étant apte à mettre en mouvement l'ensemble microbilles/mélange initial.

En particulier, l'entrée 5 est généralement reliée à une pompe péristaltique (non représentée). Cette pompe permet d'amener le mélange de départ, contenu dans le mélangeur 104, à l'intérieur de la chambre de broyage 2 via l'entrée 5. La pompe permet en plus, lors du fonctionnement du broyeur tridimensionnel, d'amener ce mélange initial selon un certain débit qui est réglable, appelé par la suite « débit de passage ». Ce débit de passage forme en outre un courant dans la chambre de broyage 2 permettant d'entraîner le mélange initial de l'entrée 5 vers la sortie 6.

La sortie 6 de la chambre de broyage 2 comprend en particulier le système de séparation 7 des microbilles du mélange final. Ce moyen de séparation 7 peut être un tamis dont les orifices présentent une dimension inférieure à celle des microbilles ou une fente de séparation dont la largeur est également adaptée pour retenir les microbilles au sein de la chambre 2.

La paroi interne 9 de la chambre de broyage 2 comprend selon un premier mode de réalisation une surface interne lisse. Cependant, selon une variante de réalisation qui sera décrite ci-après, il peut être aménagé sur cette surface interne 9 des doigts 11.

Tel que mentionné ci-dessus, à l'intérieur de la chambre de broyage 2 est disposé l'agitateur 8 qui, en plus du débit de passage, permet également la mise en mouvement du mélange initial.

En particulier, l'agitateur 8 est apte à tourner autour de l'axe X via un arbre rotatif (14, figure 3) pour impartir au sein de la chambre de broyage 2 un mouvement tourbillonnaire au mélange initial et effectuer ainsi un brassage intense entre ce mélange initial et les microbilles présentes dans la chambre 2 le long de la paroi interne 9 de cette chambre 2.

Afin d'améliorer ce brassage, l'agitateur 8, tout comme la paroi interne 9 de la chambre 2, peuvent présenter diverses configurations possibles représentées par exemple sur la figure 3.

Selon une première configuration illustrée à la figure 3a, l'agitateur 8 comprend le long de sa tige allongée des disques 10, disposés perpendiculairement à celle-ci. Leur nombre peut varier de 2 à 8, de préférence de 2 à 5. Ces disques 10 permettent d'une part, d'améliorer le broyage de la suspension de départ en brassant davantage les microbilles et d'autre part, d'accélérer le temps de réaction.

Selon une deuxième configuration illustrée à la figure 3b, l'agitateur 8 peut également comprendre le long de sa tige un ou plusieurs disques 10 disposés perpendiculaires et qui sont en outre aptes à coopérer avec des doigts 11, disposés perpendiculairement, par rapport à la paroi interne 9 de la chambre 2. Un doigt se présente notamment sous la forme d'un anneau qui s'étend perpendiculairement à partir de la paroi 9. Pour cette configuration, les disques 10 et les doigts 11 sont disposés en quinconce, à savoir les disques 10 et les doigts 11 sont disposés de manière alternée dans la chambre 2. En outre, l'épaisseur de la tige 8 est augmentée par rapport à la configuration précédente (figure 3a) de sorte que la périphérie des disques 10 soit proche de la paroi interne 9 et que celle des doigts 11 soit proche de la périphérie de la tige de l'agitateur 8. Ainsi, dans cette configuration, le volume de la chambre est réduit par rapport à la configuration précédente, permettant par conséquent, un meilleur brassage entre le mélange initial, les microbilles et la paroi interne 9 de la chambre 2.

Le volume de la chambre 2 peut encore être réduit comme cela est illustré en figure 3c. Dans cette configuration, l'agitateur 8 présente un diamètre externe légèrement inférieur au diamètre interne de la chambre 2, formant ainsi une chambre annulaire 12 de faible volume disposée entre la paroi externe de l'agitateur 8 et la paroi interne 9 de la chambre 2. Les microbilles (non représentées) sont disposées dans cette chambre annulaire 12. Lors du fonctionnement de cette configuration, le mélange initial est introduit par l'entrée 5 avec un certain débit, qui va ensuite parcourir la chambre annulaire 12 jusqu'à la sortie 6 tout en étant brassée par les microbilles.

En général, le broyeur convenant pour réaliser le procédé selon l'invention comprend une chambre de broyage présentant un diamètre de 75 mm à 300 mm pour une longueur de 80 mm à 900 mm et un agitateur présentant une taille allant de 65 mm à 260 mm. Ainsi, le volume de la chambre de broyage varie de 0,35 L à 600 L, de préférence de 0,35 L à 62 L.

La géométrie de la chambre de broyage et de l'agitateur pourra être ajustée par l'homme du métier en fonction de la quantité d'esters alkyliques d'acides gras et de glycérol souhaitée, ainsi que du temps de réaction désiré. Par exemple, il est également possible que la chambre de broyage 2 comprenne un accélérateur afin d'améliorer le broyage du le mélange initial.

En outre, les microbilles logées dans la chambre de broyage 2 et convenant pour le procédé selon l'invention sont substantiellement de forme sphérique et présentent un diamètre moyen allant de 0,05 mm à 4 mm, de préférence de 0,2 à 3 mm, en particulier de 0,3 à 2 mm, et typiquement de l'ordre de 0,5 à 1 mm. De préférence, le diamètre des microbilles est inférieur ou égal à 1 mm.

Elles sont préférentiellement choisies parmi des microbilles présentant une dureté élevée et résistant relativement bien à l'abrasion.

En particulier, les microbilles présentent une dureté de Vickers mesurée selon la norme EN ISO 6507-1 supérieure ou égale à 900 HV1, de préférence allant de 900 HV1 à 1600 HV1, typiquement allant de 1000 à 1400 HV1.

Avantageusement, elles présentent une masse volumique réelle élevée. En général, les microbilles selon l'invention ont une masse volumique réelle supérieure ou égale 2 g/cm³, en particulier allant de 2 à 15 g/cm³, de préférence de 3 à 12 g/cm³, et typiquement de 4 à 10 g/cm³.

Ainsi, les microbilles selon l'invention peuvent être des microbilles en céramique, (oxyde de zirconium ZrO₂, en silicate de zirconium ZrSiO₄) ; des microbilles en acier, des microbilles en carbure de tungstène, des microbilles en verre ou une de leurs combinaisons.

De préférence, les microbilles sont en céramiques car elles ne génèrent pas de pollution par leur usure.

En particulier, les microbilles sont en oxyde de zirconium.

Eventuellement, les microbilles en oxyde de zirconium peuvent être stabilisées par un autre oxyde, tel que l'oxyde de cérium, l'oxyde d'yttrium et/ou le silicium.

A titre d'exemples, les compositions suivantes, résumées dans le tableau 1 ci-dessous, conviennent pour former les microbilles selon l'invention :

**Tableau 1**

| **Composition des microbilles** | **Dureté HV1** | **Masse volumique réelle (g/cm³)** | **Fabricant** |
|---|---|---|---|
| Microbilles en oxyde de zirconium stabilisées par l'oxyde de cérium | 1180 | ≥ 6,10 | Saint-Gobain (Zirmil®Y Ceramic Beads) ou EIP (Procerox® ZO Cer) |
| - 80% de ZrO₂ | | | |
| - 20% de CeO | | | |
| Microbilles en oxyde de zirconium stabilisées par l'yttrium | 1250 | ≥ 5,95 | EIP (Procerox® ZO (Y)) |
| - 95% ZrO₂ | | | |
| - <5% Al₂O₃ | | | |
| - Reste : Y₂O₃ | | | |
| Microbilles en oxyde de zirconium stabilisées par l'yttrium et du silicium : | > 700 | >4,80 | Saint-Gobain (ER120 Ceramic Beads) |
| - 78% ZrO₂, | | | |
| - 12% SiO₂, | | | |
| - 5% Al₂O₃ et | | | |
| - 4% Y₂O₃ | | | |
| Microbilles en silicate de zirconium ZrSiO₄ | ≥ 800 | > 6,5 | Saint-Gobain (Rimax Ceramic Beads) |
| Microbilles en verre | 500 | > 3,76 | - |
| Microbilles en acier | 700 | > 7,7 | - |

En particulier, les microbilles représentent, en volume, par rapport au volume total de la chambre stationnaire 2 de 50% à 85%, de préférence de 55% à 70%.

A titre d'exemple, le broyeur tridimensionnel à microbilles en phase humide convenant pour réaliser le procédé selon l'invention peut correspondre à des broyeurs commercialisés par les sociétés WAB, gamme Dyno-Mill : Multi Lab, ECM et KD, Société NETZCH, par exemple LABSTAR LS1, ou encore Alpine Hosokawa, par exemple, Agitated Media Mill AHM.

Lors de l'étape de broyage (B), le mélange initial ne subit pas de préférence de traitement thermique. En effet, la température du mélange initial est inférieure ou égale à 50°C, de préférence inférieure ou égale à 35°C et typiquement inférieure ou égale à 25°C.

Tel que mentionné ci-dessus, le temps de séjour du mélange initial au sein du broyeur est très court, et est en général inférieur à 5 min. Ainsi, le procédé selon l'invention présente l'avantage de synthétiser des esters alkyliques d'acides gras en un temps très court, notamment en comparaison avec les procédés classiques de l'art antérieur.

A la sortie du broyeur 105, on récupère lors de l'étape (C) le mélange final comprenant majoritairement des esters alkyliques d'acide gras et minoritairement du glycérol, un excédent de monoalcool aliphatique (alcool qui n'a pas réagi), ainsi que le catalyseur.

En général, ce mélange final comprend, en masse, par rapport à sa masse totale :
- de 60% à 95%, de préférence de 65% à 92%, et typiquement de 67 % à 90% d'esters alkyliques d'acides gras
- de 0% à 30%, de préférence de 0.5% à 25%, et typiquement de 1% à 20% de monoalcool aliphatique qui n'a pas réagi (excédent)
- de 2,5% à 25%, de préférence de 5% à 20%, et typiquement de 7% à 15%, d'un mélange contenant du glycérol et le catalyseur, ces deux composés, pouvant ou non, et ce de manière partielle ou non, réagir ensemble pour former un troisième composé.

Le mélange final peut également contenir des traces d'autres composants, comme des glycérides partiellement convertis (monoglycérides, diglycérides et triglycérides), ainsi que des traces d'eau et d'ions métalliques, notamment des ions alcalins.

Les constituants qui sont non-miscibles, tels que les esters alkyliques d'acides gras, le glycérol et le catalyseur, sont ensuite séparés lors de l'étape (D) qui a lieu dans un séparateur 106.

En effet, dans ce mélange final, le glycérol pur a une masse volumique proche de 1,2 g.cm⁻³, alors que celle des esters alkyliques d'acides gras se situe autour de 0,9 g.cm⁻³. En présence de peu de méthanol, la phase contenant majoritairement du glycérol (G) est donc plus dense que la phase ester (H) et a ainsi tendance à se placer en dessous de cette dernière sous l'effet de la gravité. La phase ester constitue donc la phase surnageante lorsqu'on procède à la séparation. En général, le monoalcool n'ayant pas réagi se retrouve, tout comme le catalyseur, avec le glycérol au fond du séparateur 106, sous forme d'une phase visqueuse, hétérogène.

Ce séparateur 106 est bien connu de l'homme du métier et peut correspondre à tout dispositif apte à séparer la phase supérieure comprenant les esters alkyliques d'acides gras (H) et la phase inférieure (G) comprenant le glycérol qui est récupéré dans un conteneur 109. A titre d'exemple, le séparateur 106 peut être un décanteur à contre-courant, un séparateur centrifuge, tel qu'un hydrocyclone, un séparateur membranaire ou encore un ballon décanteur.

Cette étape s'effectue également sans chauffage particulier, à savoir à une température inférieure ou égale à 50°C, de préférence inférieure ou égale à 35°C et typiquement inférieure ou égale à 25°C.

Généralement, le rendement de la réaction de transestérification à l'issue de l'étape (D) est supérieur ou égal à 95 %, mieux supérieur ou égal à 98%, et encore mieux supérieur ou égal à 99%.

De préférence, le procédé comprend en outre une étape (E) d'élimination du monoalcool avant et/ou après l'étape (D) de séparation. Le monoalcool contenu dans le mélange final en sortie du broyeur tridimensionnel à billes 105 ou à la sortie du séparateur 106 (à savoir se trouvant dans la phase glycérol) peut être recyclé. Cette étape de recyclage est également bien connue de l'homme du métier et pourra correspondre à une étape d'évaporation/condensation du monoalcool telle que décrite dans l'art antérieur.

Egalement, le catalyseur pourra être recyclé lors d'une étape (F) selon une technique bien connue de l'homme du métier. Il pourra par exemple subir un ou plusieurs lavages et ou être traité thermiquement avant d'être stocké ou réintroduit dans le processus.

Enfin, la phase d'esters alkyliques d'acide gras (H) issu du séparateur 106 peut subir une étape de traitement (I) de façon notamment à répondre à la spécification concernant la teneur en glycérine totale (libre et liée) de la norme EN 14214 : 2013 relative au biodiesel. Ce traitement (I) peut être un ou plusieurs passages sur une résine échangeuse d'ions (108), pouvant être par exemple de la Purolite® PD206, dans le cas présent pour piéger l'ion calcium.

Ce traitement de l'ester brut peut se faire de différentes façons via divers dispositifs 110.

Par exemple, on peut éventuellement faire passer l'ester dans un moyen de purification qui élimine les dernières traces de glycérine libre insoluble (par exemple par passage dans un coalesceur), et/ou la glycérine dissoute par exemple sur des masses adsorbantes, telles que des résines échangeuses d'ions, dans un adsorbeur. Le traitement de l'ester peut, dans d'autres cas, se faire grâce à une ou plusieurs étapes de lavage à l'eau de l'ester ou tout simplement par filtration.

La présente invention peut porter également sur un produit susceptible d'être obtenu par le procédé susmentionné, comprenant au moins des esters d'acides gras, du glycérol, un catalyseur et un monoalcool, caractérisé en ce qu'il comporte moins de 40%, de préférence moins de 30 % molaire, par rapport à la stoechiométrie totale du produit, dudit monoalcool.

De préférence, le monoalcool, tel que le méthanol, représente, de 0,05 à 20% molaire par rapport à la quantité totale de produit.

L'invention a également pour objet un produit susceptible d'être obtenu par le procédé susmentionné, comprenant au moins des esters d'acides gras, du glycérol, un catalyseur et un monoalcool, caractérisé en ce qu'il comporte moins de 30 %, en masse, par rapport à la masse totale du produit, dudit monoalcool.

Bien évidemment les caractéristiques décrites pour le procédé selon l'invention telles que définies ci-dessus, sont également valables pour le présent produit. Elles ne seront donc pas décrites plus en détail ci-après.

### EXEMPLES

La description des essais ci-dessous est donnée à titre d'exemple purement illustratif et non limitatif. Sauf indications contraires, les pourcentages sont donnés en masse.

### A° Caractérisation chromatographie en phase liquide à haute performance HPLC

Les essais de chromatographie HPLC ont été réalisés en utilisant un chromatographe JASCO équipé d'une pompe à gradient quaternaire PU-208, d'un détecteur multi longueur d'onde (MD-2015), d'un passeur d'échantillon (AS-2055° et d'un four à colonne (co-2065) utilisant une colonne à phase inversée PHENOMENEX LUNA C18 (250 mm × 4.6 mm, 5 µm).

Les solvants ont été filtrés à travers un filtre de 0,45 mm et dégazés avec de l'hélium. Un gradient linéaire de 100% méthanol à 50% méthanol + 50% de 2-propanol-hexane (5 :4, v/v) en 35 minutes a été employé.

Le volume d'injection est de 15 mL et le débit de 1 mL/min.

La température de la colonne a été maintenue constante à 40°C.

Tous les échantillons ont été dissous dans le 2-propanol-hexane (5 :4, v/v).

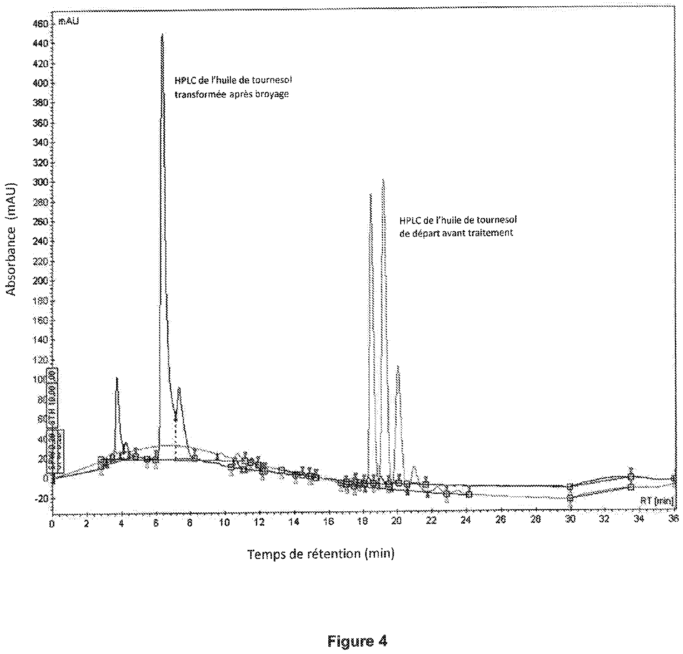

La teneur massique nommée FAME (figure 4) déterminée par HPLC est considérée comme représentant le rendement massique en % des esters alkyliques d'acides gras obtenus par le procédé selon l'invention. Les pics des esters alkyliques sont identifiées entre 6 et 8 minutes, ceux des mono et ou diglycérides entre 10 et 15 minutes et enfin ceux des triglycérides entre 18 et 22 minutes. Cette figure correspond à l'analyse de la référence n° 3 présentée dans le tableau 3 ci-dessous

### B° Procédure de préparation des échantillons testés

### ✔Appareillage

Les essais ont été mis en oeuvre dans un broyeur tridimensionnel à microbilles Dyno Mill MultiLab de la Société Willy A. Bachofen AG qui contient 1 KG de microbilles.

Les microbilles sont en oxyde de zirconium et présentent un diamètre de 500 µm. Elles sont notamment commercialisées sous le nom de marque Zirmil® Y Ceramic Beads par la société Saint-Gobain.

La chambre de broyage du broyeur présente une capacité de 309 mL et est remplie, en volume, par rapport à son volume total et en fonction des essais, de 80% des microbilles décrites ci-dessus.

En fonctionnement, les microbilles sont mises en agitation par un agitateur à une vitesse de rotation de 2890 tr/min. L'agitateur comporte en outre deux disques mélangeurs en polyuréthane de 64 mm de diamètre.

### ✔ Matières premières

Pour les essais, les matières premières de départ sont :

**Tableau 2**

| Matières premières | Nom commercial | Référence | Caractéristiques |
|---|---|---|---|
| Huile | | | |
| l'huile de tournesol | Huile de tournesol raffinée | Olvea N° de lot 12596 | wintérisée conforme à la Ph.Eur en vigueur-Tonnelet 55kg |
| l'huile de soja | Huile de soja raffinée identité préservée | Olvea N° de lot 12280 | conforme à la Ph.Eur en vigueur- Tonnelet 28kg |
| l'huile de palme | Huile de palme raffinée | Olvea N° de lot 12553 | carton 10kg |
| huiles usées | Huile de tournesol | | Filtrée et déshydratée |

| Catalyseur | | | |
|---|---|---|---|
| KOH | Hydroxyde de potassium 90% | Mon droguiste.com | Conditionnée en sac 1 kg |
| Diglycéroxyde de calcium | Cadg Diglycéroxyde de calcium | Easyl SA | Pureté > 99% |
| Zincate de calcium | ZnO-CaO 99% | Easyl SA | Calciné sous air à 400 °C |

| Matières premières | Nom commercial | Référence | Caractéristiques |
|---|---|---|---|
| Monoalcool | | | |
| Méthanol | 99.9% | PRODUITS CHIMIQUES PLATRET | |

En particulier, le catalyseur hétérogène de zincate de calcium a été préparé en suivant le procédé suivant décrit notamment dans le brevet FR 15 52884 :
- une suspension de départ est préparée dans un Bécher à partir de l'hydroxyde de calcium (74 g) et de l'oxyde de zinc (162.8 g), en proportion stoechiométrique, dans de l'eau déminéralisée, soit à une concentration « produits de départ/eau déminéralisée» de 300g/L; puis la suspension de départ est mise sous agitation à l'aide d'un agitateur magnétique ;
- elle est ensuite amenée, via une pompe péristaltique à débit réglable au broyeur Dyno Mill MultiLab décrit ci-dessus : les débits de passage testés dans le broyeur sont de 30l/h correspondant à des temps de séjour respectifs de 8 s ;
- la suspension de départ est ensuite broyée dans le broyeur comportant des microbilles de 0,5 mm de diamètre pendant une certaine durée (qui dépend comme indiqué ci-dessus du débit de passage de la suspension de départ) à température ambiante (20-25°C), permetant ainsi, à la sortie du broyeur, l'obtention d'une suspension de cristaux de zincate de calcium ;
- enfin, la suspension de cristaux de zincate de calcium est récupérée et calcinée à 400°C pendant 60 min

### ✔ Procédure générale mise en oeuvre pour les essais :

Pour réaliser chaque essai ci-dessous, les étapes suivantes sont réalisées :
- le catalyseur a été pré-mélangé au méthanol lorsque celui-ci était homogène (KOH) ou à l'huile lorsqu'il était hétérogène (zincate de calcium, diglyceroxyde de calcium) ;
- l'huile, le catalyseur et le méthanol ont été ensuite mélangé dans un mélangeur mécanique à hélices pendant 1 min à température ambiante (∼20-25°C) de sorte à obtenir un mélange initial ;
- une fois homogénéisé, ce mélange initial a été introduit dans le broyeur décrit ci-dessus à température ambiante (20-25°C) selon les paramètres indiqués dans le tableau 3 ci-après, de façon à obtenir un mélange final (un passage) ;
- ce mélange final a ensuite été décanté dans une ampoule à décanter pendant 24 heures à température ambiante (20-25°C);
- les échantillons obtenus ont ensuite été analysés par HPLC, de sorte notamment à déterminer le % de conversion massique FAME.

### C° Résultats

Comme le montre le tableau 3 ci-après, le procédé selon l'invention permet d'obtenir d'excellents taux de conversion en esters méthyliques d'acides gras (taux de conversion %FAME supérieur ou égal à 95%) et ce, en étant facile à mettre en oeuvre, tout en étant rapide (temps de séjour inférieur à 5 min dans le broyeur) et en ne requérant pas d'étapes de chauffage ou de mise sous pression particulière.

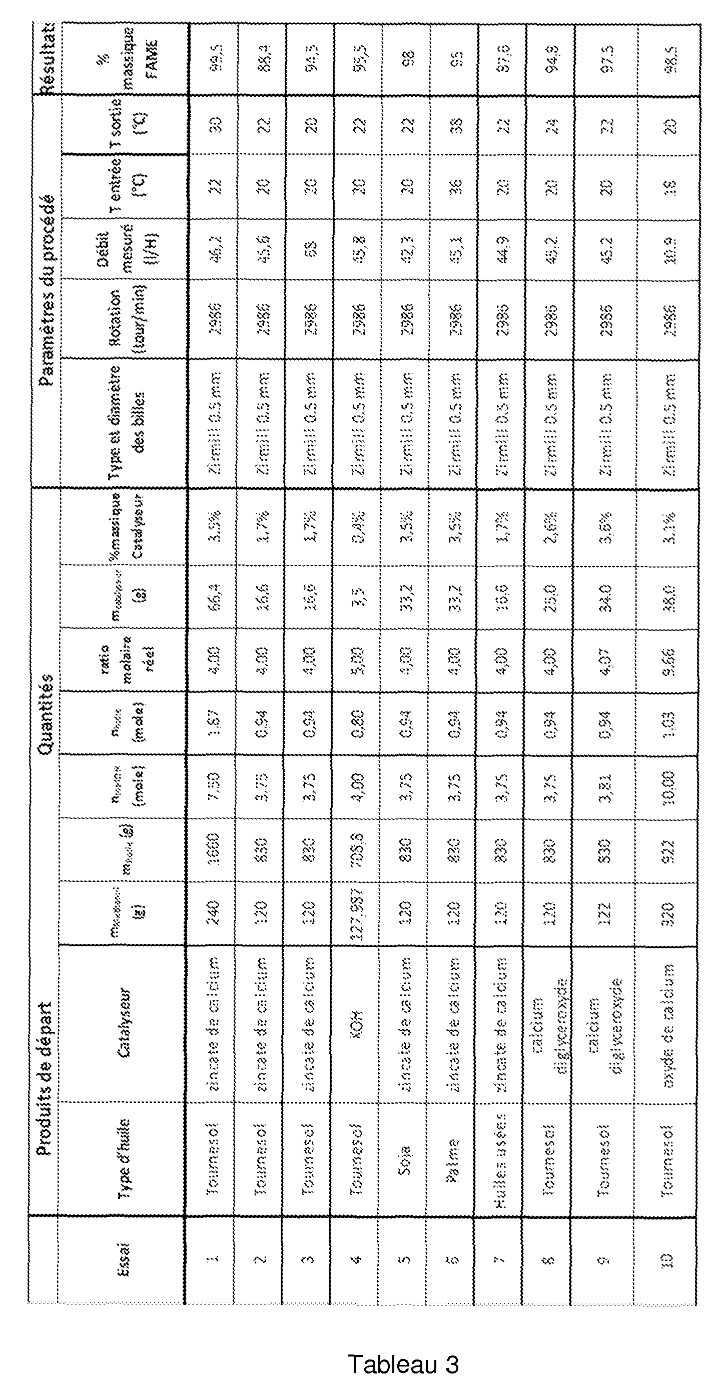

## Revendications

1. Procédé de production d'esters alkyliques d'acides gras et de glycérol mettant en oeuvre un ensemble de réactions de transestérification entre au moins une huile végétale ou animale et au moins un monoalcool aliphatique comprenant les étapes suivantes :
(A) l'introduction dans un broyeur tridimensionnel à microbilles séparément ou en mélange d'au moins une huile végétale et/ou animale, d'au moins un monoalcool aliphatique et d'au moins un catalyseur hétérogène et/ou homogène pour former un mélange initial ;
(B) le broyage dudit mélange initial à une température inférieure ou égale à 50°C, de préférence inférieure ou égale à 25°C, dans un broyeur tridimensionnel à microbilles, pendant un temps de séjour inférieur ou égal à 5 minutes, de préférence allant de 5 à 30 secondes et typiquement allant de 5 à 15 secondes ;
(C) la récupération en sortie du broyeur tridimensionnel d'un mélange final comportant au moins des esters alkyliques d'acides gras, du glycérol, le catalyseur et le monoalcool aliphatique n'ayant pas réagi ; et
(D) la séparation de ce mélange final d'une première phase comprenant les esters alkyliques d'acides gras et d'une deuxième phase comprenant le glycérol, le monoalcool aliphatique n'ayant pas réagi et le catalyseur.

2. Procédé selon la revendication 1, **caractérisé en ce qu'**il comprend en outre une étape (E) d'élimination du monoalcool avant ou après l'étape (D) de séparation.

3. Procédé selon la revendication 1 ou 2, **caractérisé en ce que** le rapport molaire monoalcool/huile dans le mélange initial est inférieur à 15, de préférence inférieur à 6 et mieux égal ou inférieur à 4, sans jamais être inférieur à 3.

4. Procédé selon l'une des revendications 1 à 3, **caractérisé en ce que** le rapport massique catalyseur/huile+monoalcool dans le mélange initial est inférieur à 5%, de préférence inférieur à 3% et typiquement égal à 2%.

5. Procédé selon l'une des revendications 1 à 4, **caractérisé en ce que** le mélange initial a une teneur en eau inférieure à 1500 ppm.

6. Procédé selon l'une des revendications 1 à 5, **caractérisé en ce que** le monoalcool aliphatique est choisi parmi un ou plusieurs des monoalcools suivants: le méthanol, l'éthanol, le propanol, l'isopropyle ou encore le butanol, de préférence le monoalcool est le méthanol ou l'éthanol.

7. Procédé selon l'une des revendications 1 à 6, **caractérisé en ce que** le catalyseur est un catalyseur hétérogène solide ou un catalyseur homogène liquide.

8. Procédé selon la revendication 7, **caractérisé en ce que** le catalyseur hétérogène solide est choisi parmi un ou plusieurs des composés suivants : l'oxyde de calcium, l'oxyde de zinc, un mélange d'oxyde de zinc et d'alumine, un aluminate de zinc répondant à la formule ZnAl₂O₄, (ZnO)ₓ (Al₂O₃)_{y} où x et y sont compris chacun entre 0 et 2, le zincate de calcium et le calcium diglycéroxyde.

9. Procédé selon la revendication 8, **caractérisé en ce que** le catalyseur hétérogène solide est du zincate de calcium de formule Ca[Zn(OH)₃]₂.2H₂O, préalablement calciné à une température allant de 400°C à 600 °C.

10. Procédé selon la revendication 7, **caractérisé en ce que** le catalyseur homogène est choisi parmi les hydroxydes et méthoxydes des métaux alcalins et alcalinoterreux et les acides, tels que H₂ SO₄.

11. Procédé selon l'une quelconque des revendications précédentes, **caractérisé en ce qu'**il comprend une étape de récupération et de recyclage du catalyseur dans le procédé.

12. Procédé selon l'une des revendications 1 à 11, **caractérisé en ce que** le rendement de la réaction de transestérification à l'issue de l'étape (D) est supérieur ou égal à 95%, mieux supérieur ou égal à 98%.

13. Procédé selon l'une des revendications 1 à 12, **caractérisé en ce que** le broyeur tridimensionnel à microbilles comprend au moins :
- une chambre de broyage stationnaire de forme générale cylindrique s'étendant selon un axe longitudinal XX, ladite chambre étant remplie au moins, en partie, par lesdites microbilles et comprend: à une première extrémité au moins une entrée servant à introduire ladite suspension de départ, et à une seconde extrémité, une sortie comportant un moyen de séparation apte à n'évacuer que le mélange final ainsi formé dans ladite chambre ; et
- un agitateur, disposé dans la chambre de broyage stationnaire, se présentant sous forme d'une tige allongée selon l'axe longitudinal XX, ledit agitateur étant apte à mettre en mouvement l'ensemble microbilles/suspension de départ.

14. Procédé selon la revendication 13, dans lequel les microbilles représentent, en volume, par rapport au volume total de la chambre stationnaire de 50% à 85%, de préférence de 55% à 70%.

## Patentansprüche

1. Verfahren zur Herstellung von Alkylestern von Fettsäuren und Glycerin unter Verwendung einer Reihe von Umesterungsreaktionen zwischen mindestens einem pflanzlichen oder tierischen Öl und mindestens einem aliphatischen Monoalkohol, umfassend die folgenden Schritte:
(A) Einbringen von mindestens einem pflanzlichen und/oder tierischen Öl, mindestens einem aliphatischen Monoalkohol und mindestens einem heterogenen und/oder homogenen Katalysator in eine dreidimensionale Mikrokugelmühle, getrennt oder gemischt, um ein Ausgangsgemisch zu bilden;
(B) Mahlen des Ausgangsgemisches bei einer Temperatur von 50 °C oder weniger, vorzugsweise 25 °C oder weniger, in einer dreidimensionalen Mikrokugelmühle während einer Verweilzeit von 5 Minuten oder weniger, vorzugsweise 5 bis 30 Sekunden und typischerweise 5 bis 15 Sekunden;
(C) Rückgewinnen einer Endmischung aus der dreidimensionalen Mühle, die mindestens Alkylester von Fettsäuren, Glycerin, Katalysator und nicht umgesetzten aliphatischen Monoalkohol enthält; und
(D) Trennen dieser Endmischung von einer ersten Phase, die die Alkylester von Fettsäuren umfasst, und einer zweiten Phase, die Glycerin, nicht umgesetzten aliphatischen Monoalkohol und Katalysator umfasst.

2. Verfahren nach Anspruch 1, **dadurch gekennzeichnet, dass** es ferner einen Schritt (E) zum Entfernen des Monoalkohols vor oder nach dem Schritt (D) des Trennens umfasst.

3. Verfahren nach Anspruch 1 oder 2, **dadurch gekennzeichnet, dass** das Molverhältnis von Monoalkohol zu Öl in der Ausgangsmischung weniger als 15, vorzugsweise weniger als 6 und besser 4 oder weniger, aber nie weniger als 3 beträgt.

4. Verfahren nach einem der Ansprüche 1 bis 3, **dadurch gekennzeichnet, dass** das Massenverhältnis von Katalysator/Öl + Monoalkohol im Ausgangsgemisch weniger als 5 %, vorzugsweise weniger als 3 % und typischerweise gleich 2 % beträgt.

5. Verfahren nach einem der Ansprüche 1 bis 4, **dadurch gekennzeichnet, dass** das Ausgangsgemisch einen Wassergehalt von weniger als 1500 ppm aufweist.

6. Verfahren nach einem der Ansprüche 1 bis 5, **dadurch gekennzeichnet, dass** der aliphatische Monoalkohol ausgewählt ist aus einem oder mehreren der folgenden Monoalkohole; Methanol, Ethanol, Propanol, Isopropyl oder Butanol, wobei es sich bei dem Monoalkohol vorzugsweise um Methanol oder Ethanol handelt.

7. Verfahren nach einem der Ansprüche 1 bis 6, **dadurch gekennzeichnet, dass** es sich bei dem Katalysator um einen festen heterogenen Katalysator oder einen flüssigen homogenen Katalysator handelt.

8. Verfahren nach Anspruch 7, **dadurch gekennzeichnet, dass** der feste heterogene Katalysator ausgewählt ist aus einer oder mehreren der folgenden Verbindungen: Calciumoxid, Zinkoxid, einer Mischung aus Zinkoxid und Aluminiumoxid, einem Zinkaluminat der Formel ZnAl₂O₄, (ZnO)ₓ (Al₂O₃)_{y}, wobei x und y jeweils zwischen 0 und 2 liegen, Calciumzinkat und Calciumdiglyceroxid.

9. Verfahren nach Anspruch 8, **dadurch gekennzeichnet, dass** der feste heterogene Katalysator Calciumzinkat der Formel Ca[Zn(OH)₃]₂.2H₂O ist, das zuvor bei einer Temperatur im Bereich von 400 °C bis 600 °C kalziniert wurde.

10. Verfahren nach Anspruch 7, **dadurch gekennzeichnet, dass** der homogene Katalysator ausgewählt ist aus Hydroxiden und Methoxiden von Alkali- und Erdalkalimetallen und Säuren, wie beispielsweise H₂SO₄.

11. Verfahren nach einem der vorstehenden Ansprüche, **dadurch gekennzeichnet, dass** es einen Schritt zum Rückgewinnen und Wiederverwerten des Katalysators in dem Verfahren umfasst.

12. Verfahren nach einem der Ansprüche 1 bis 11, **dadurch gekennzeichnet, dass** die Ausbeute der Umesterungsreaktion am Ende von Schritt (D) 95 % oder mehr, besser 98 % oder mehr beträgt.

13. Verfahren nach einem der Ansprüche 1 bis 12, **dadurch gekennzeichnet, dass** die dreidimensionale Mikrokugelmühle mindestens umfasst:
eine stationäre Mahlkammer von allgemein zylindrischer Form, die sich entlang einer Längsachse XX erstreckt, wobei die Kammer zumindest teilweise mit den Mikrokugeln gefüllt ist und umfasst: an einem ersten Ende mindestens einen Einlass zum Einführen der Ausgangssuspension und an einem zweiten Ende einen Auslass mit einem Trennmittel, das geeignet ist, nur das so in der Kammer gebildete Endgemisch abzuführen; und
- ein Rührwerk, das in der stationären Mahlkammer in Form einer länglichen Stange entlang der Längsachse XX angeordnet ist, wobei das Rührwerk in der Lage ist, die Ausgangsanordnung Mikrokugeln/Suspension in Bewegung zu setzen.

14. Verfahren nach Anspruch 13, wobei die Mikrokugeln, bezogen auf das Gesamtvolumen der stationären Kammer, ein Volumen von 50 % bis 85 %, vorzugsweise 55 % bis 70 %, ausmachen.

## Claims

1. Method for producing fatty acid alkyl esters and glycerol, implementing a set of transesterification reactions between at least one vegetable or animal oil and at least one aliphatic monohydric alcohol comprising the following steps:
(A) introducing in a three-dimensional microbead mill separately or as a mixture of at least one vegetable and/or animal oil, at least one aliphatic monohydric alcohol and at least one heterogenous and/or homogenous catalyst to form an initial mixture;
(B) grinding said initial mixture at a temperature less than or equal to 50°C, preferably less than or equal to 25°C, in a three-dimensional microbead mill, for a residence time less than or equal to 5 minutes, preferably going from 5 to 30 seconds and typically going from 5 to 15 seconds;
(C) recovering, at the outlet of the three-dimensional mill, a final mixture comprising at least fatty acid alkyl esters, glycerol, the catalyst and the aliphatic monohydric alcohol not having reacted; and
(D) separating this final mixture from a first phase comprising fatty acid alkyl esters and from a second phase comprising glycerol, the aliphatic monohydric alcohol not having reacted and the catalyst.

2. Method according to claim 1, **characterised in that** it further comprises a step (E) of removing the monohydric alcohol before or after the separation step (D).

3. Method according to claim 1 or 2, **characterised in that** the monohydric alcohol/oil molar ratio in the initial mixture is less than 15, preferably less than 6, and best equal to or less than 4, without ever being less than 3.

4. Method according to one of claims 1 to 3, **characterised in that** the catalyst/oil + monohydric alcohol mass ratio in the initial mixture is less than 5%, preferably less than 3% and typically equal to 2%.

5. Method according to one of claims 1 to 4, **characterised in that** the initial mixture has a water content less than 1500 ppm.

6. Method according to one of claims 1 to 5, **characterised in that** the aliphatic monohydric alcohol is selected from among one or more of the following monohydric alcohols: methanol, ethanol, propanol, isopropyl or butanol, preferably the monohydric alcohol is methanol or ethanol.

7. Method according to one of claims 1 to 6, **characterised in that** the catalyst is a solid heterogenous catalyst or a liquid homogenous catalyst.

8. Method according to claim 7, **characterised in that** the solid heterogenous catalyst is selected from among one or more of the following compounds: calcium oxide, zinc oxide, an zinc oxide and alumina mixture, a zinc aluminate responding to the formula ZnAl₂O₄ (ZnO)ₓ (Al₂O₃)_{y} where x and y are each between 0 and 2, calcium zincate and calcium diglyceroxide.

9. Method according to claim 8, **characterised in that** the solid heterogenous catalyst is calcium zincate of the formula Ca[Zn(OH)₃]₂.2H₂O, calcined beforehand at a temperature going from 400°C to 600°C.

10. Method according to claim 7, **characterised in that** the homogenous catalyst is selected from among hydroxides and methoxides of alkaline and alkaline earth metals and acids, such as H₂SO₄.

11. Method according to any of the preceding claims, **characterised in that** it comprises a step of recovering and recycling the catalyst in the method.

12. Method according to one of claims 1 to 11, **characterised in that** the return of the transesterification reaction coming from step (D) is greater than or equal to 95%, best greater than or equal to 98%.

13. Method according to one of claims 1 to 12, **characterised in that** the three-dimensional microbead mill comprises at least:
- a stationary grinding chamber of general cylindrical shape extending along a longitudinal axis XX, said chamber being filled at least partially, with said microbeads and comprises: at a first end, at least one inlet used for introducing said initial suspension, and at a second end, an outlet comprising a separation means, capable of only releasing the final mixture thus formed in said chamber; and
- a stirrer, disposed in the stationary grinding chamber, being presented in the form of an elongated rod along the longitudinal axis XX, said stirrer being capable of moving the microbeads/initial suspension unit.

14. Method according to claim 13, wherein the microbeads represent, by volume, with respect to the total volume of the stationary chamber, 50% to 85%, preferably 55% to 70%.
